# EUROPEAN PATENT APPLICATION

(11) **EP 3 425 051 A1**
(43) Date of publication of application: **09.01.2019**
(21) Application number: 17179874.7
(22) Date of filing: 05.07.2017
(51) Int. Cl.: C12P 5/00, C12N 9/10

(54) **METHOD FOR THE PRODUCTION OF ISOPRENOIDS BY HETEROLOGOUS EXPRESSION OF A PROTEIN IN A MICROORGANISM**

(71) Applicant: DECHEMA -Forschungsinstitut, 60486 Frankfurt (DE)
(72) Inventor: Buchhaupt, Markus, 60486 Frankfurt am Main (DE); Kschowak, Max, 60486 Frankfurt am Main (DE); Drummond, Laura, 60486 Frankfurt am Main (DE); Schrader, Jens, 60486 Frankfurt am Main (DE)
(74) Representative: Engelhard, Markus

(57) **Abstract**

The present disclosure relates to a method for the production of isoprenoids by heterologous expression of a protein in a microorganism.

The present disclosure relates to an alkyl transferase capable of catalyzing one or more alkylation(s) of isopentenyl-pyrophosphate.

The present disclosure also relates to an alkyl transferase capable of catalyzing the one or more methylation(s) of isopentenyl-pyrophosphate and optionally the one or more isomerization(s) of isopentenyl-pyrophosphate or alkylated isopentenyl-pyrophosphate.

The methyltransferase was obtained from Streptomyces monomycini.

SEQUENCE LISTING

<110> Dechema Forschungsinstitut

<120> Method for the production of alkylated prenyldiphosphates

<130> D31578EP

<160> 13

<170> BiSSAP 1.3.2

<210> 1
<211> 300
<212> PRT
<213> Streptomyces monomycini

<223> "IPP-methyltransferase"

<220>
<223> IPP-methyltransferase

<400> 1
Met Ser Ser Glu Pro Thr Ala Ala Leu Arg Thr Pro Leu Glu Gln Ala
1               5                   10                  15
Ala Phe Asp Tyr Tyr Asp Asn Lys Arg Asp Asp Pro Ile Asn Leu Lys
            20                  25                  30
Leu Gly Glu Thr Asp Gly Phe Tyr His His His Phe Ala Val Gly Asp
        35                  40                  45
Phe Asp Arg Lys Val Leu Leu Thr Ser Gly Glu Glu Arg Ala Gln Ala
    50                  55                  60
Ile Asn Ala Glu Leu His Arg Met Glu Thr Arg Gln Val Asp Ala Leu
65                  70                  75                  80
Ser Glu Ala Leu Gly Pro Val Ala Pro Gln Ala Arg Ile Leu Asp Gly
                85                  90                  95
Gly Ser Gly Arg Gly Gly Thr Ala Ile Leu Leu His Lys Ala Phe Gly
            100                 105                 110
Cys Arg Val Asp Gly Val Asn Ile Ser Ala Tyr Gln Asn Glu Phe Ala
        115                 120                 125
Arg Arg Gln Ala Gln Glu His Asn Cys Ala Glu Lys Val His Phe His
    130                 135                 140
Asp Arg Asn Met Ala Ala Thr Gly Phe Pro Asp Ala Ser Phe Asp Tyr
145                 150                 155                 160
Val Val Thr Asn Glu Thr Thr Met Tyr Val Asp Pro Tyr Glu Thr Phe
                165                 170                 175
Ala Glu Phe Ala Arg Leu Leu Lys Pro Ala Gly Arg Tyr Val Leu Leu
            180                 185                 190
Thr Trp Cys Asn Asn Asp Val Leu Ala Pro His Pro Pro Glu Ala Thr
        195                 200                 205
Ala Ile Asp Ala His Tyr His Cys His Thr His Arg Arg Thr Thr Tyr
    210                 215                 220
Leu Lys Ala Leu Ile Glu Ser Gly Leu Ile Pro Tyr Gln Val Asp Asp
225                 230                 235                 240
Leu Thr Gln Pro Ala Thr Pro Tyr Trp Glu Leu Arg Ser Tyr Ser Arg
                245                 250                 255
Tyr Ala Thr Gly Ile Glu Gln His Tyr Leu Asp Gly Tyr Arg Ser Asp
            260                 265                 270
Arg Val Asn Tyr Ile Arg Ile Ala Ser Arg Arg Gly Ala Val Pro Pro
        275                 280                 285
Ser Asp Thr Pro Thr Thr Ala Gly Ala Ala Ala His
    290                 295                 300

<210> 2

<211> 64
<212> DNA
<213> Artificial Sequence

<223> "primer mk34"

<220>
<223> primer mk34

<400> 2
taacaattcc cctctagata agattaaata aggaggttac caatgtcctc tgagccaact     60

gctg                                                                  64


<210> 3
<211> 36
<212> DNA
<213> Artificial Sequence

<223> "primer mk36"

<220>
<223> primer mk36

<400> 3
agctcgaatt cggatccttc agtgggcggc ggcgcc                               36


<210> 4
<211> 19
<212> DNA
<213> Artificial Sequence

<223> "primer mk32"

<220>
<223> primer mk32

<400> 4
aggatccgaa ttcgagctc                                                  19


<210> 5
<211> 45
<212> DNA
<213> Artificial Sequence

<223> "primer mk33"

<220>
<223> primer mk33

<400> 5
tggtaacctc cttatttaat cttatctaga ggggaattgt tatcc                     45


<210> 6
<211> 38
<212> DNA
<213> Artificial Sequence

<223> "primer mk42"

<220>
<223> primer mk42

<400> 6
acaggagtcc aagcgagctc aggtggcact tttcgggg                                38


<210> 7
<211> 50
<212> DNA
<213> Artificial Sequence

<223> "primer mk44"

<220>
<223> primer mk44

<400> 7
ggcaccgacg tcttaattaa aacaataaaa ctgtctgctt acataaacag                    50


<210> 8
<211> 18
<212> DNA
<213> Artificial Sequence

<223> "primer mk20"

<220>
<223> primer mk20

<400> 8
gagctcgctt ggactcct                                                      18


<210> 9
<211> 28
<212> DNA
<213> Artificial Sequence

<223> "primer mk23"

<220>
<223> primer mk23

<400> 9
ttaattaaga cgtcggtgcc taatgagt                                           28


<210> 10
<211> 23
<212> DNA
<213> Artificial Sequence

<223> "primer mk8"

<220>
<223> primer mk8

<400> 10
cctaaggatc caaactcgag taa                                                23

<210> 11
<211> 24
<212> DNA
<213> Artificial Sequence

<223> "primer mk31"

<220>
<223> primer mk31

<400> 11
tataggatcc gtgcagtcgg cgaa                                              24


<210> 12
<211> 26
<212> DNA
<213> Artificial Sequence

<223> "primer mk52"

<220>
<223> primer mk52

<400> 12
agtccatatg tcctctgagc caactg                                            26


<210> 13
<211> 20
<212> DNA
<213> Artificial Sequence

<223> "primer mk53"

<220>
<223> primer mk53

<400> 13
aattcggatc cttcagtggg                                                   20

## Description

### BACKGROUND

The field of the DISCLOSURE lies in methods for the production of isoprenoids.

The present disclosure relates to a method for the production of isoprenoids by heterologous expression of a protein in a microorganism.

The present disclosure relates to an alkyl transferase capable of catalyzing one or more alkylation(s) of isopentenyl-pyrophosphate.

The present disclosure also relates to an alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate.

The present disclosure relates to a nucleic acid encoding the alkyl transferase capable of catalyzing one or more alkylation(s) and optionally one or more isomerization(s) of isopentenyl-pyrophosphate or alkylated isopentenyl-pyrophosphate.

The present disclosure relates further to a vector containing the nucleic acid encoding the alkyl transferase, a host cell containing the nucleic acid encoding the alkyl transferase or the vector containing the nucleic acid encoding the alkyl transferase.

Moreover, the present disclosure relates to isoprenoids produced by the methods for the production of isoprenoids according to the present disclosure.

### DESCRIPTION OF THE RELATED ART

From isoprenoids many natural substances of interest can be derived. Examples thereof are steroids, essential oils, rubber or pharmaceuticals like taxol or artemisinin. The direct precursor and starting point of the biosynthesis of the isoprenoids is not isoprene, which can be seen in Scheme 1 below, 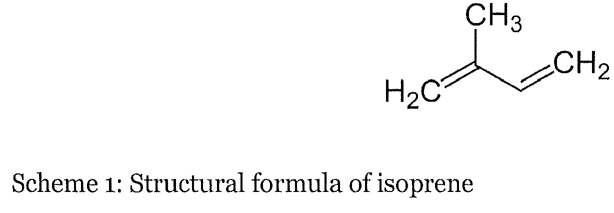 but the biochemically activated isopentenyl pyrophosphate (IPP) and its isomer dimethylallyl pyrophosphate (DMAPP), which can both be seen in Schemes 2 and 3 below. 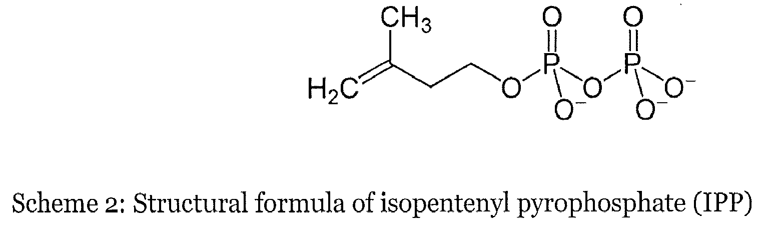 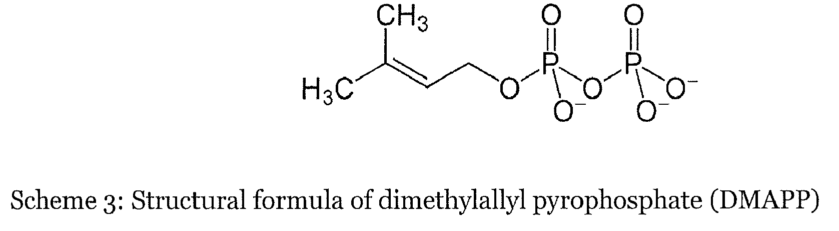 Isoprene itself is produced by many trees and phytoplankton.

Terpenes are a large and diverse class of organic compounds, produced by a variety of plants, particularly conifers, bacteria, fungi or animals. For example, some insects such as termites or swallowtail butterflies emit terpenes from their osmeteria. Terpenes often have a strong odor and may protect the plants that produce them by deterring herbivores and by attracting predators and parasites of herbivores. The difference between terpenes and terpenoids is that terpenes are hydrocarbons, whereas terpenoids contain additional functional groups.

Terpenes are the major components of resin and of turpentine produced from resin. In addition to their roles as end-products in many organisms, terpenes are major biosynthetic building blocks within nearly every living creature. Steroids, for example, are derivatives of the triterpene squalene.

When terpenes are modified chemically, such as by oxidation or rearrangement of the carbon skeleton, the resulting compounds are generally referred to as terpenoids. Sometimes the term terpene is used to include all terpenoids. Terpenoids are also known as isoprenoids.

Terpenes and terpenoids are the primary constituents of the essential oils of many types of plants and flowers. Essential oils are used widely as fragrances in perfumery, and in medicine and alternative medicines such as aromatherapy. Synthetic variations and derivatives of natural terpenes and terpenoids also greatly expand the variety of aromas used in perfumery and flavors used in food additives.

Terpenes are structurally derived from units of isoprene, which has the molecular formula C₅H₈. The basic molecular formulae of terpenes are multiples of (C₅H₈)ₙ, where n is the number of linked isoprene units. This is called the isoprene rule or the C₅-rule according to Ruzicka and Wallach. Due to this rule, it is hardly possible to obtain terpenes, which do not subordinate to this rule. Other molecule-sizes for terpenes than (C₅H₈)ₙ can be obtained when additional, further, in most cases enzymatic, reactions are carried out after the terpene synthesis, so that molecule-parts are splitted off or when alkylations are carried out with the help of enzymes. However, such enzymes are very rare.

Isoprene itself does not undergo the building process, but rather the activated forms isopentenyl-pyrophosphate (IPP or also isopentenyl-diphosphate) and dimethylallyl-pyrophosphate (DMAPP or also dimethylallyl-diphosphate, which is an isomer of IPP) are the components in the biosynthetic pathway. IPP is formed from acetyl-CoA via the intermediacy of mevalonic acid in the mevalonate pathway. An alternative, totally unrelated biosynthesis pathway of IPP is known in some bacterial groups and the plastids of plants, the so-called MEP (2-Methyl-D-erythritol-4-phosphate)-pathway, which is initiated from C₅-sugars. In both pathways, IPP is isomerized to DMAPP by the enzyme isopentenyl-pyrophosphate isomerase.

During terpene synthesis, the C₅-molecules isopentenyl-pyrophosphate (IPP) and dimethylallyl-pyrophosphate (DMAPP) are condensed to longer prenyldiphosphates. Afterwards, various terpene synthases build one or more products with longer molecule sizes based on IPP, DMAPP, geranyl-pyrophosphate (GPP, C₁₀), farnesyl-pyrophosphate (FPP, C₁₅), geranylgeranyl-pyrophosphate (GGPP, C₂₀) or other prenyldiphosphates.

As chains of isoprene units are built up thereby, the resulting terpenes are classified sequentially by size as hemiterpenes, monoterpenes, sesquiterpenes, diterpenes, sesterterpenes, triterpenes, tetraterpenes and polyterpenes. Essentially, terpene synthases are the enzymes necessary for conversion of the respective prenyldiphosphates into terpenes or terpenoids in most cases.

In the following, a scheme of the biosynthesis of terpenoids is shown, which leads to the synthesis of terpenes as described above:

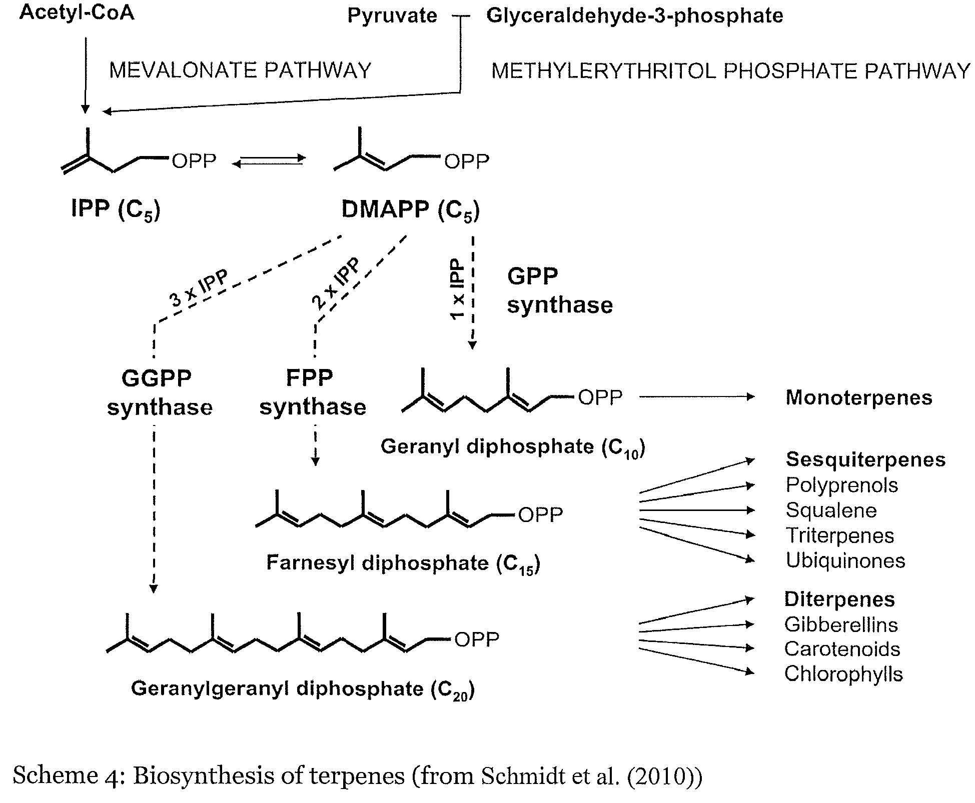

After synthesis, the isoprene units as a general rule are linked together "head to tail" to form chains, as can be seen in Scheme 4 above. One can consider the isoprene unit as one of nature's common building blocks.

The above described isoprene rule is however not valid for some bacterial terpenes. Further, for example, the biosynthesis of 2-methylisoborneol, which is a C₁₁-compound, also has been described according to Komatsu et al. (2008), Dickschat et al. (2007) and Wang et al. (2008). The respective reaction scheme therefore is shown in the following Scheme 5:

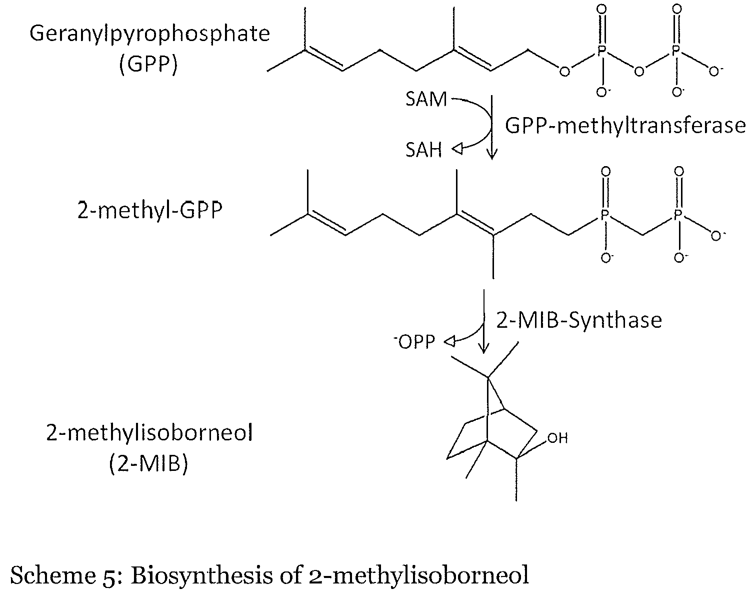

Further, a geranyl-pyrophosphate-methyl transferase according to Köksal et al. (2012) is known.

However, the need for further proteins for producing isoprenoids, which do not obey to the isoprene rule is still of enormous relevance.

### SUMMARY

The present disclosure provides a method for the production of isoprenoids by heterologous expression of a protein in a microorganism,
wherein, in said method, a protein is heterologously expressed as an alkyl transferase, wherein the protein is able to catalyze one or more alkylation(s) of isopentenyl-pyrophosphate.

The present disclosure further provides a method for the production of isoprenoids by heterologous expression of a protein in a microorganism,
wherein, in said method, the protein is heterologously expressed as an alkyl transferase and as an isomerase,
wherein the protein is able to catalyze, additionally to the one or more alkylation(s) of isopentenyl-pyrophosphate, one or more isomerization(s) of isopentenyl-pyrophosphate or of alkylated isopentenyl-pyrophosphate.

The present disclosure further provides a method for the production of isoprenoids by heterologous expression of a protein in a microorganism,
wherein, in said method, the protein is heterologously expressed as an alkyl transferase and as an isomerase,
wherein the protein is able to catalyze, additionally to the one or more alkylation(s) of isopentenyl-pyrophosphate, one or more isomerization(s) of alkylated isopentenyl-pyrophosphate.

The present disclosure further provides an alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate, said alkyl transferase having an amino acid sequence represented by SEQ ID NO: 1. The present disclosure further provides an alkyl transferase capable of catalyzing the one or more alkylation(s) of isopentenyl-pyrophosphate and optionally the one or more isomerization(s) of alkylated isopentenyl-pyrophosphate, said alkyl transferase having an amino acid sequence represented by SEQ ID NO: 1.

The present disclosure also provides one or several isoprenoids produced by the method according to the present disclosure for the production of isoprenoids by heterologous expression of a protein in a microorganism, wherein, in said method, a protein is heterologously expressed as an alkyl transferase and optionally as an isomerase, wherein the protein is able to catalyze one or more alkylation(s) of isopentenyl-pyrophosphate and optionally one or more isomerization(s) of isopentenyl-pyrophosphate or alkylated isopentenyl-pyrophosphate, preferably one or more isomerization(s) of alkylated isopentenyl-pyrophosphate.

The foregoing paragraphs have been provided by way of general introduction, and are not intended to limit the scope of the following claims. The described embodiments, together with further advantages, will be best understood by reference to the following detailed description taken in conjunction with the accompanying drawings.

### BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:

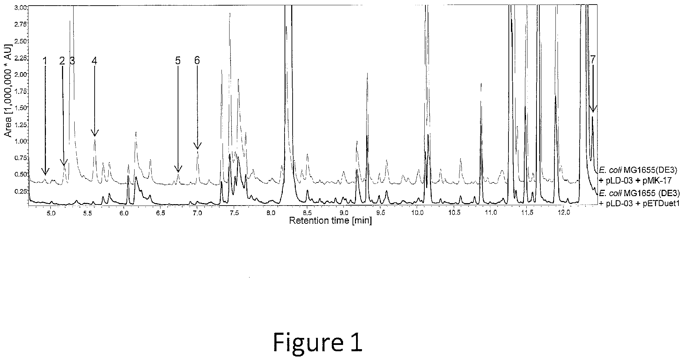

Figure 1 is a total-ion-chromatogramm of HS-SPME-GC/MS analysis of cultures of *E. coli* MG1655(DE3)+ pLD-o3 + pMK-17 and *E. coli* MG1655 (DE3)+ pLD-o3 + pETDuet1.

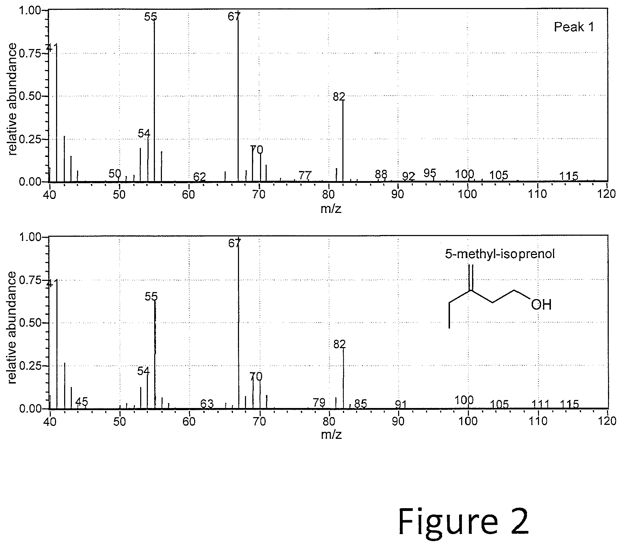

Figure 2 is a peak corresponding mass spectra of 5-methyl-isoprenol compared to the mass spectra of the standard compound 5-methyl-isoprenol.

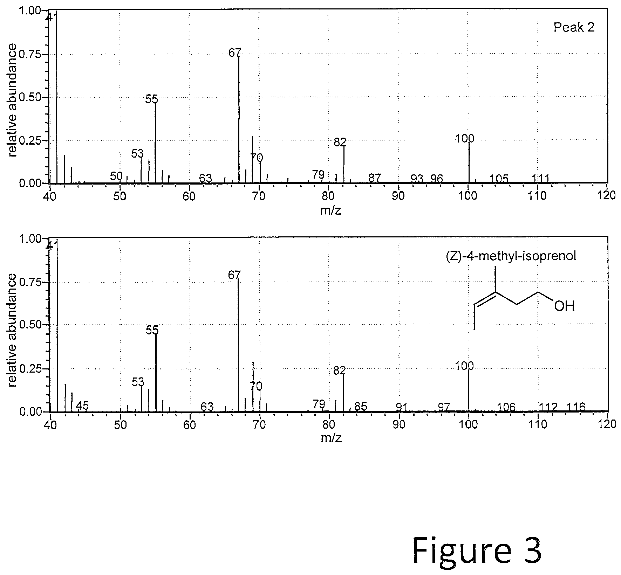

Figure 3 is a peak corresponding mass spectra of (Z)-4-methyl-isoprenol compared to the mass spectra of the standard compound (Z)-4-methyl-isoprenol.

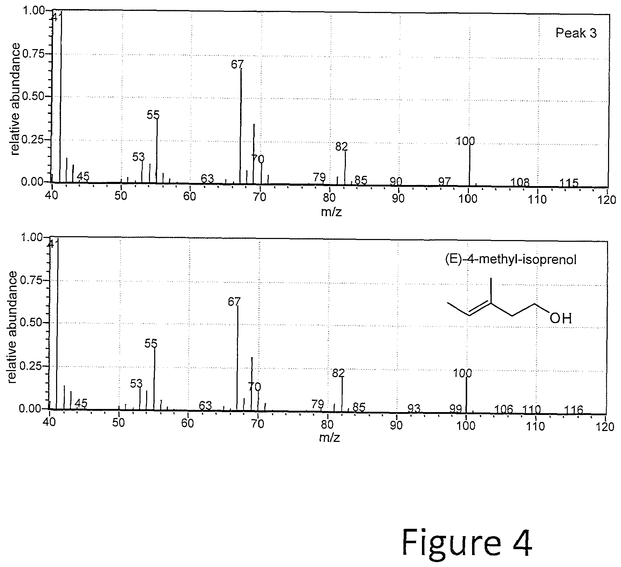

Figure 4 is a peak corresponding mass spectra of (E)-4-methyl-isoprenol compared to the mass spectra of the standard compound (E)-4-methyl-isoprenol.

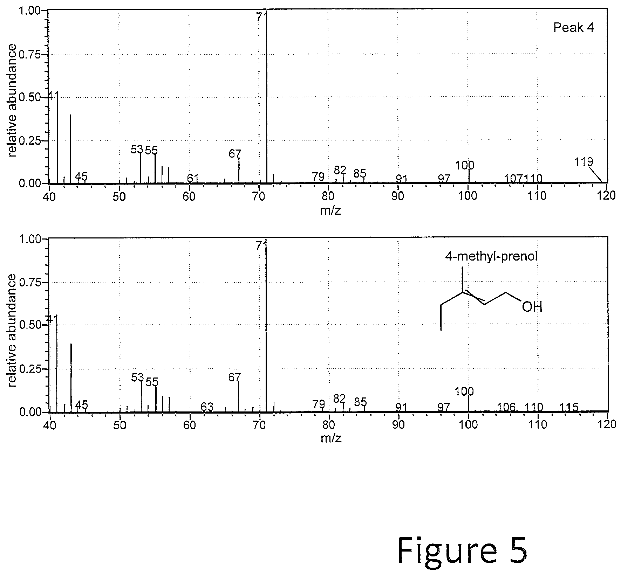

Figure 5 is a peak corresponding mass spectra of 4-methyl-prenol compared to the mass spectra of the standard compound 4-methyl-prenol.

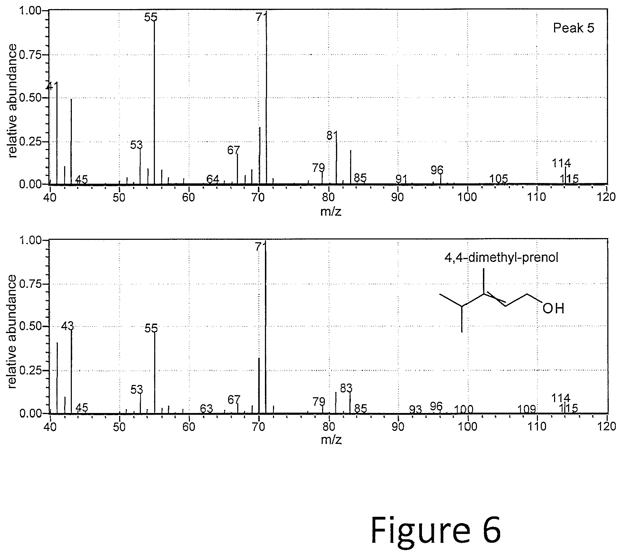

Figure 6 is a peak corresponding mass spectra of 4,4-dimethyl-prenol compared to the mass spectra of the standard compound 4,4-dimethyl-prenol.

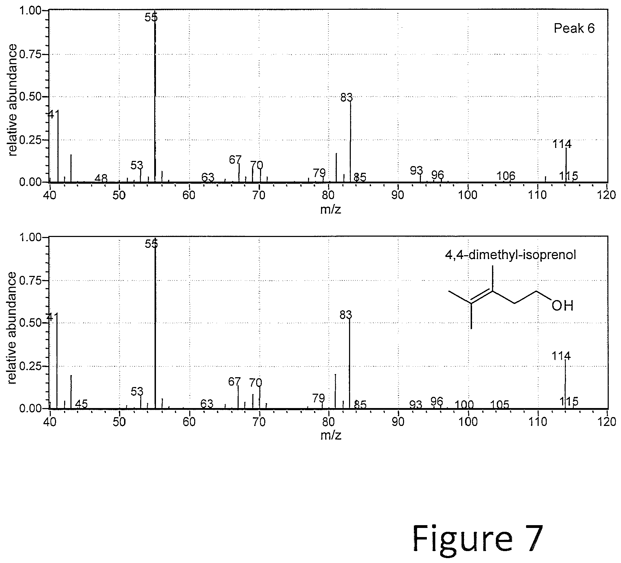

Figure 7 is a peak corresponding mass spectra of 4,4-dimethyl-isoprenol compared to the mass spectra of the standard compound 4,4-dimethyl-isoprenol.

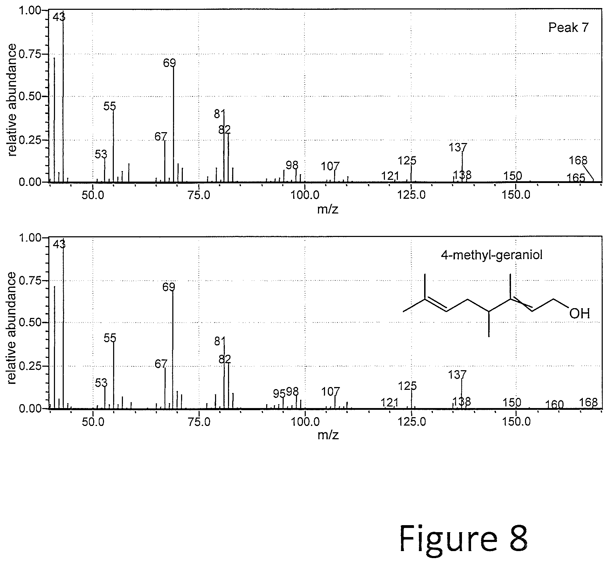

Figure 8 is a peak corresponding mass spectra of 4-methyl-geraniol compared to the mass spectra of the standard compound 4-methyl-geraniol.

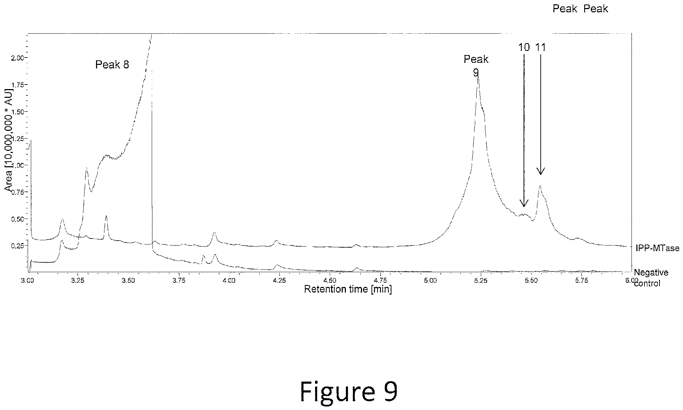

Figure 9 is a chromatogram giving proof of the presence of isoprenol (peak 8), (Z)-4-methyl-isoprenol (peak 9), (E)-4-methyl-isoprenol (peak 10) and 4-methyl-prenol (peak 11) in an in *vitro* assay.

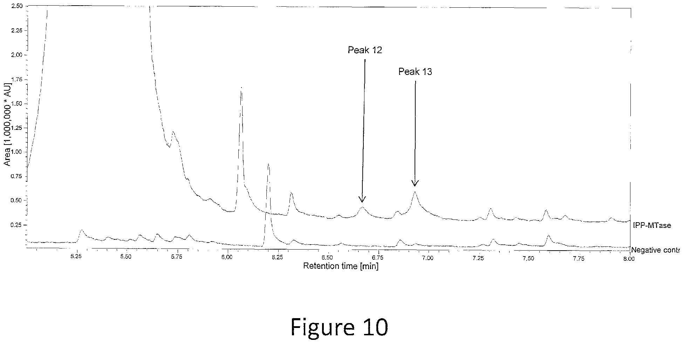

Figure 10 shows the same chromatogram of an in *vitro* assay as Figure 9, however, the scaling has been amended for a better display of the peaks of 4,4-dimethyl-prenol (peak 12) and 4,4-dimethyl-isoprenol (peak 13).

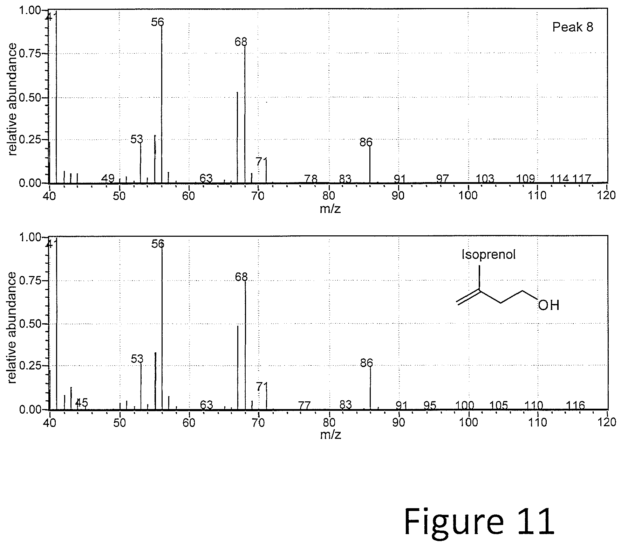

Figure 11 is a peak corresponding mass spectra in an *in vitro* assay of isoprenol compared to the mass spectra of the standard compound isoprenol.

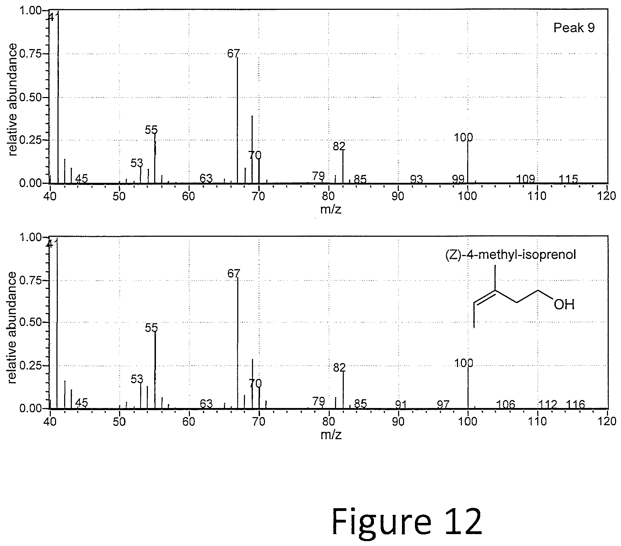

Figure 12 is a peak corresponding mass spectra in an in *vitro* assay of (Z)-4-methyl-isoprenol compared to the mass spectra of the standard compound (Z)-4-methyl-isoprenol.

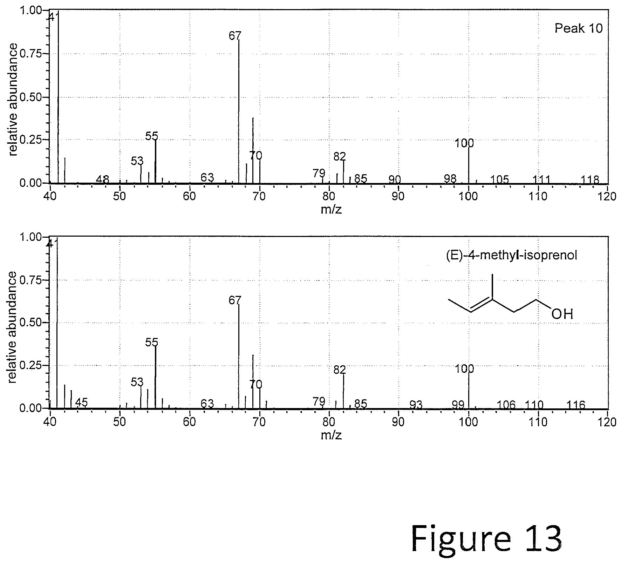

Figure 13 is a peak corresponding mass spectra in an *in vitro* assay of (E)-4-methyl-isoprenol compared to the mass spectra of the standard compound (E)-₄-methyl-isoprenol.

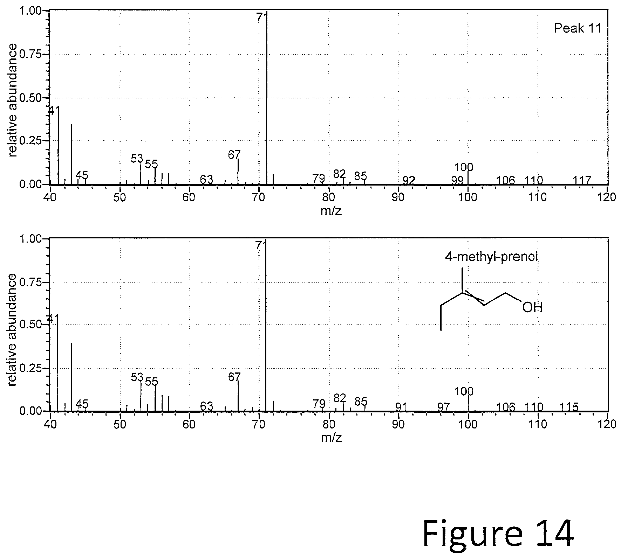

Figure 14 is a peak corresponding mass spectra in an in *vitro* assay of 4-methyl-prenol compared to the mass spectra of the standard compound 4-methyl-prenol.

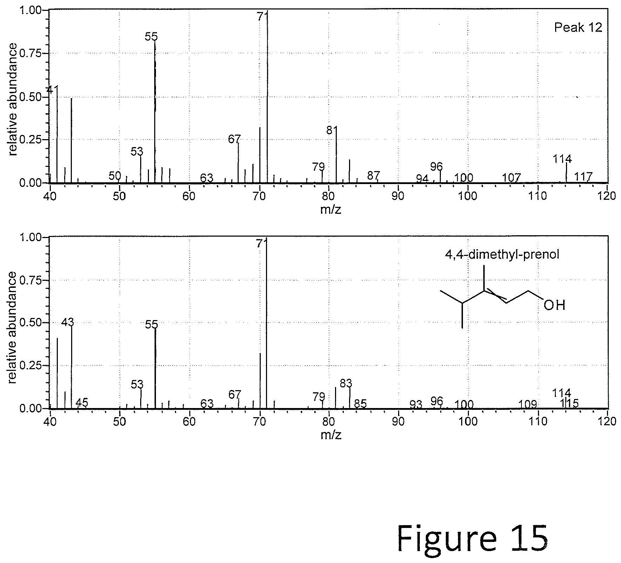

Figure 15 is a peak corresponding mass spectra in an in *vitro* assay of 4,4-dimethyl-prenol compared to the mass spectra of the standard compound 4,4-dimethyl-prenol.

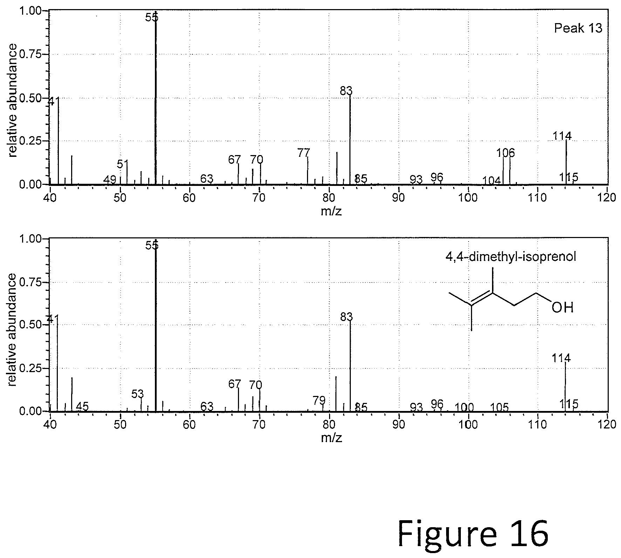

Figure 16 is a peak corresponding mass spectra in an in *vitro* assay of 4,4-dimethyl-isoprenol compared to the mass spectra of the standard compound 4,4-dimethyl-isoprenol.

### DETAILED DESCRIPTION OF THE EMBODIMENTS

As discussed above, the present disclosure provides methods for the production of isoprenoids by heterologous expression of a protein in a microorganism.

The method for the production of isoprenoids according to the present disclosure is a method for the production of isoprenoids by heterologous expression of a protein in a microorganism,
wherein, in said method, a protein is heterologously expressed as an alkyl transferase, wherein the protein is able to catalyze one or more alkylation(s) of isopentenyl-pyrophosphate.

An alkyl transferase is any transferase enzyme that enacts the transfer of alkyl groups, which can be, for example, a methyl-, ethyl-, propyl-, butyl- or pentyl-group, from one molecule to another. "Alkylation", as used herein, therefore refers to the transfer of one or several of any of the aforementioned groups. In one embodiment, alkylation is preferably a methylation.

For example, the expressed alkyl transferase can be able to catalyze the methylation of isopentenyl-pyrophosphate according to the following reaction scheme 6:

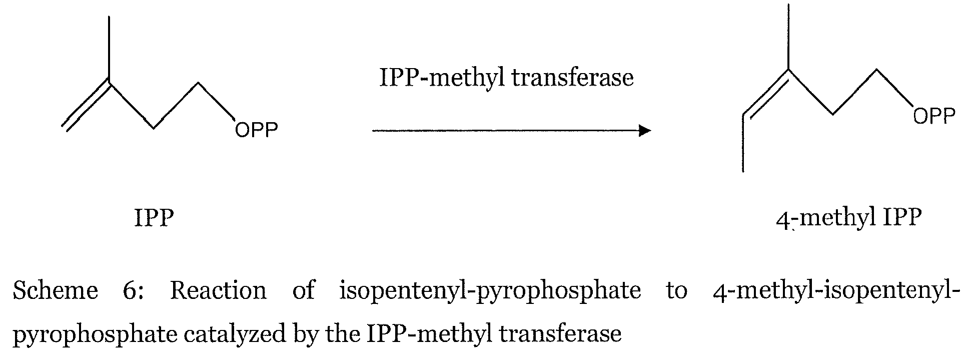

In an embodiment of the method for the production of isoprenoids by heterologous expression of a protein in a microorganism according to the present disclosure, the protein is heterologously expressed as an alkyl transferase and as an isomerase, wherein the protein is able to catalyze, additionally to the one or more alkylation(s) of isopentenyl-pyrophosphate, one or more isomerization(s) of isopentenyl-pyrophosphate or of alkylated isopentenyl-pyrophosphate. Thus, the expressed alkyl transferase is not only able to catalyze one or more alkylation(s) of isopentenyl-pyrophosphate, but also able to catalyze one or more alkylation(s) and one or more isomerization(s) of isopentenyl-pyrophosphate or of alkylated isopentenyl-pyrophosphate.

It is preferred for this embodiment, that the protein is able to catalyze, additionally to the one or more alkylation(s) of isopentenyl-pyrophosphate, one or more isomerization(s) of alkylated isopentenyl-pyrophosphate.

Isomerization is the process, by which one molecule is transformed into another molecule, which has exactly the same atoms, but the atoms have a different arrangement. Thus, for example, the heterologously expressed protein of the method of the present disclosure is not only able to catalyze the methylation of isopentenyl-pyrophosphate to e.g. 4-methyl-isopentenyl-pyrophosphate, but also the isomerization of 4-methyl-isopentenyl-pyrophosphate to 4-methyl-dimethylallyl-pyrophosphate.

This means that the protein being heterologously expressed in the method of the disclosure described herein, is in any case an alkyl transferase, however, can additionally serve as an isomerase of the alkylated product(s) of isopentenyl-pyrophosphate.

In an embodiment, in the method according to the present disclosure, wherein a protein is heterologously expressed as alkyl transferase and optionally as an isomerase, the heterologous expression is in Escherichia coli, Saccharomyces cerevisiae, Pichia pastoris, Bacillus subtilis, Corynebacterium glutamicum, Pfaffia rhodozyma, Methylobacterium extorquens, Pseudomonas putida, Cupriavidus necator, Rhodobacter sphaeroides, Streptomyces avermitilis, Streptomyces coelicolor or in Streptomyces monomycini.

The term "a protein is heterologously expressed", as used herein, refers to a heterologous expression of such protein. The term "a protein is expressed", as used herein, refers to any expression of such protein, which can be heterologous or homologous. Further, if, in the present disclosure, a protein is said to be expressed as an enzyme, e.g. as an alkyl transferase, this is used synonymously with a scenario, wherein such enzyme, e.g. alkyl transferase, is expressed.

In an embodiment, in the method according to the present disclosure, wherein a protein is heterologously expressed as alkyl transferase and optionally as isomerase, the heterologous expression is in Escherichia coli.

In one embodiment, the protein is an alkyl transferase. In one embodiment, the protein is an alkyl transferase and an isomerase. In one embodiment, the protein comprises an alkyl transferase and an isomerase activity. In one embodiment, the protein is characterized by having an amino acid sequence given by SEQ ID NO. 1. In one embodiment, the protein is expressed as an isopentenyl-pyrophosphate-methyl transferase. In one embodiment, the protein is expressed as an isopentenyl-pyrophosphate-methyl transferase, wherein the isopentenyl-pyrophosphate-methyl transferase is an alkyl transferase and an isomerase. In one embodiment, the protein is expressed as an isopentenyl-pyrophosphate-methyl transferase, wherein the isopentenyl-pyrophosphate-methyl transferase comprises an alkyl transferase and an isomerase activity. In one embodiment, the protein is expressed as an isopentenyl-pyrophosphate-methyl transferase given by the SEQ ID NO. 1. In one embodiment, the protein is expressed as an isopentenyl-pyrophosphate-methyl transferase given by the SEQ ID NO. 1, wherein the isopentenyl-pyrophosphate-methyl transferase given by the SEQ ID NO. 1 is an alkyl transferase and an isomerase. In one embodiment, the protein is expressed as an isopentenyl-pyrophosphate-methyl transferase given by the SEQ ID NO. 1, wherein the isopentenyl-pyrophosphate-methyl transferase given by the SEQ ID NO. 1 comprises an alkyl transferase and an isomerase activity.

In an embodiment, in the method according to the present disclosure, wherein a protein is expressed as alkyl transferase and optionally as isomerase, at least a further protein additionally to the alkyl transferase is expressed, which is able to catalyze the condensation of two or more substrates selected from the group consisting of isopentenyl-pyrophosphates, dimethylallyl-pyrophosphates, geranyl-pyrophosphates, farnesyl-pyrophosphates and geranylgeranyl-pyrophosphates, wherein at least one substrate of this further protein is an alkylated isopentenyl-pyrophosphate, an alkylated dimethylallyl-pyrophosphate, an alkylated geranyl-pyrophosphate, an alkylated farnesyl-pyrophosphate or an alkylated geranylgeranyl-pyrophosphate.

This means that, for example, in the method described herein, a protein is expressed as an alkyl transferase but also as an isomerase, so that this protein is able to catalyze the methylation of isopentenyl-pyrophosphate to 4-methyl-isopentenyl-pyrophosphate, but is also able to catalyze the isomerization of 4-methyl-isopentenyl-pyrophosphate , to 4-methyl-dimethylallyl-pyrophosphate. Such an alkylated dimethylallyl-pyrophosphate can then be used as a substrate for the condensation of two or more substrates as described in the paragraph above.

In an embodiment, in the method according to the present disclosure, wherein a protein is heterologously expressed as alkyl transferase and optionally as isomerase, one or more other protein(s) additionally to the alkyl transferase is or are expressed, which is one or more protein(s) selected from the group consisting of phosphatase(s), isomerase(s), prenyltransferase(s) and synthase(s), wherein at least one substrate of the one or more protein(s) is an alkylated isopentenyl-pyrophosphate, an alkylated dimethylallyl-pyrophosphate, an alkylated geranyl-pyrophosphate, an alkylated farnesyl-pyrophosphate or an alkylated geranylgeranyl-pyrophosphate. Thus, an alkylated dimethylallyl-pyrophosphate can also be gained by an additionally expressed isomerase, which is different from the heterologously expressed alkyl transferase of the method described herein, but also by the heterologously expressed alkyl transferase of the method described herein, which can have an isomerase-activity.

A phosphatase enzyme in general catalyzes the hydrolysis of its substrate and is thus a subcategory of hydrolases. Phosphatase enzymes catalyze the addition or hydrolysis of one or more phosphomonoester(s) or phosphodiester(s) or of more than two phosphogroups. Thus, phosphatase enzymes are essential to a myriad of biological functions, because phosphorylation and dephosphorylation serve diverse roles in cellular regulation and signaling. In detail, when for example dephosphorylation of one phosphomonoester takes place, water is split in such reactions, with the -OH-group attaching to the phosphate ion, and the H⁺ protonating the hydroxyl group of the other build product. Thus, in the simplest case, the net result of a reaction of a phosphatase enzyme is the destruction of a phosphomonoester and the creation of a phosphate ion and a molecule with a free hydroxyl group. A phosphatase enzyme as used in the present disclosure is preferably an enzyme that catalyses the hydrolysis of phosphodiesters.

In an embodiment, the expressed phosphatase enzyme(s) according to the present disclosure is one or more isopentenyl-pyrophosphatase.

Isomerases are a general class of enzymes which convert a molecule from one isomer to another. Isomerases can either facilitate intramolecular rearrangements, in which bonds are broken and formed. Thus, isomerases catalyze changes within one molecule. They convert one isomer to another, meaning that the end product has the same molecular formula but a different physical structure.

In an embodiment, the expressed isomerase enzyme(s) according to the present disclosure, which is expressed additionally to the heterologously expressed alkyl transferase, which can also serve as an isomerase, is one or more isopentenyl-pyrophosphate-isomerase. An isopentenyl-pyrophosphate-isomerase isomerizes, for example, 4-methyl-isopentenyl-pyrophosphate reversible to 4-methyl-dimethylallylpyrophosphate according to the following reaction scheme 7:

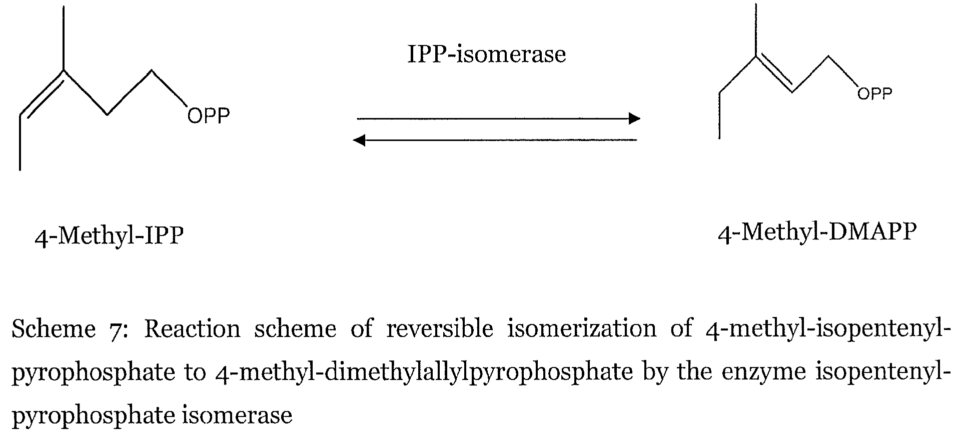

A synthase is an enzyme that catalyses a synthesis process. Following the EC number classification, they belong to the group of ligases, with lyases catalysing the reverse reaction. According to the originally, biochemical nomenclature, it is distinguished between synthetases and synthases. Under the original definition, synthases do not use energy from nucleoside triphosphates (such as ATP, GTP, CTP, TTP, and UTP), whereas synthetases do. However, the Joint Commission on Biochemical Nomenclature (JCBN) dictates that "synthase" can be used with any enzyme that catalyzes synthesis (whether or not it uses nucleoside triphosphates), whereas "synthetase" is to be used synonymously with "ligase". In the present disclosure the term "synthase" is to be understood as any enzyme that catalyzes a synthesis process, preferably the synthesis of a terpene molecule.

In a preferred embodiment, a synthase enzyme according to the present disclosure is one or more terpene synthase or one or more terpene cyclase. A terpene synthase according to the present disclosure is any enzyme that catalyzes the synthesis of terpenes and a terpene cyclase according to the present disclosure is any enzyme that catalyzes a chemical reaction to form cyclic terpene(s).

In another embodiment, the one or more other protein, which is/are expressed additionally to the alkyl transferase, which can also serve as an isomerase, is one or more prenyltransferase(s). A prenyltransferase is an enzyme that transfers allylic prenyl groups to acceptor molecules, in the case of the present disclosure to terpene(s). Prenyl transferases commonly refer to prenyl diphosphate synthases.

In an embodiment, the expressed prenyltransferase according to the present disclosure is a 4-methyl-geranyl-pyrophosphate-synthase.

In an embodiment, in the method according to the present disclosure, wherein a protein is heterologously expressed as alkyl transferase and optionally as isomerase, the one or more other protein(s), which is or are expressed additionally to the alkyl transferase, is expressed as an isopentenyl-pyrophosphate-isomerase, which is able to catalyze the reversible isomerisation of 4-methyl-isopentenyl-pyrophosphate to 4-methyl-dimethylallyl-pyrophosphate.

In an embodiment, in the method according to the present disclosure, wherein a protein is heterologously expressed as alkyl transferase and optionally as isomerase, the one or more other protein(s), which is or are expressed additionally to the alkyl transferase, is expressed as an isopentenyl-pyrophosphate-isomerase, which is able to catalyze the reversible isomerisation of 4-methyl-isopentenyl-pyrophosphate to 5-methyl-isopentenyl-pyrophosphate.

In an embodiment, in the method according to the present disclosure, wherein a protein is heterologously expressed as alkyl transferase and optionally as isomerase, the one or more other protein(s), which is expressed additionally to the alkyl transferase, is expressed as a 4-methyl-geranyl-pyrophosphate-synthase.

The present disclosure further provides an alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate, said alkyl transferase having an amino acid sequence represented by SEQ ID NO: 1. Preferably, the present disclosure further provides an alkyl transferase capable of catalyzing the one or more alkylation(s) of isopentenyl-pyrophosphate and optionally the one or more isomerization(s) of alkylated isopentenyl-pyrophosphate, said alkyl transferase having an amino acid sequence represented by SEQ ID NO: 1.

The present disclosure further provides a nucleic acid encoding the alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate, preferably the one or more isomerization(s) of alkylated isopentenyl-pyrophosphate.

The present disclosure further provides a vector containing the nucleic acid encoding the alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate, preferably the one or more isomerization(s) of alkylated isopentenyl-pyrophosphate.

The present disclosure further provides a host cell containing the nucleic acid encoding the alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate, preferably the one or more isomerization(s) of alkylated isopentenyl-pyrophosphate, or the vector containing the nucleic acid encoding the alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate, preferably the one or more isomerization(s) of alkylated isopentenyl-pyrophosphate.

The present disclosure further provides isoprenoids produced by any of the methods described above for the production of isoprenoids by heterologous expression of a protein in a microorganism,
wherein, in said method, a protein is heterologously expressed as an alkyl transferase and optionally as an isomerase, wherein the protein is able to catalyze one or more alkylation(s) of isopentenyl-pyrophosphate and optionally one or more isomerization(s) of isopentenyl-pyrophosphate or alkylated isopentenyl-pyrophosphate, preferably one or more isomerization(s) of alkylated isopentenyl-pyrophosphate.

In a preferred embodiment, the one or more isoprenoid(s) produced by any of the methods according to the present disclosure for the production of isoprenoids by heterologous expression of a protein in a microorganism is an isoprenoid selected from the group consisting of:
5-Methylisoprenol (according to IUPAC: 3-Ethylbut-3-en-1-ol) 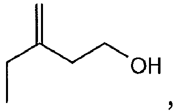
(Z)-4-methylisoprenol (according to IUPAC: (Z)-3-Methyl-3-penten-1-ol) 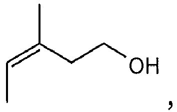
(E)-4-methylisoprenol (according to IUPAC: (E)-3-Methyl-3-penten-1-ol) 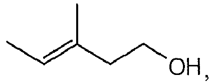
4-methylprenol (according to IUPAC: 3-Methyl-2-penten-1-ol) 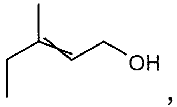
4,4-dimethylprenol (according to IUPAC: 3,4-Dimethyl-2-penten-1-ol) 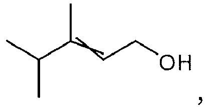
4,4-dimethylisoprenol (according to IUPAC: 3,4-Dimethyl-3-penten-1-ol) 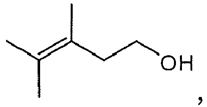 and
4-methyl-geraniol (according to IUPAC: 3,4,7-Trimethyl-2,6-octadien-1-ol)

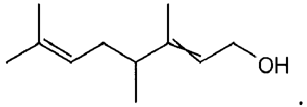

### EXAMPLES

The alkyl transferase, which is able to catalyze one or more alkylation(s) of isopentenyl-pyrophosphate and optionally one or more isomerization(s) of isopentenyl-pyrophosphate or alkylated isopentenyl-pyrophosphate according to the present disclosure is characterized in the following:

The alkyl transferase, which can additionally serve as an isomerase, according to the present disclosure is in this Example an isopentenyl-pyrophosphate-methyl transferase, which transfers a methyl-group to isopentenyl-pyrophosphate so that methylated isopentenyl-pyrophosphate, namely 4-methyl-isopentenyl-pyrophosphate, is synthesized.

### Plasmid construction

The constructions of plasmids were done via Gibson assembly (Gibson et al., 2009) or standard restriction-cloning. pMK-17 including the gene of the IPP-methyl transferase with SEQ ID NO: 1 was constructed via Gibson assembly. The insert was amplified from the genomic DNA of *S. monomycini* (DSM No. 41801) via PCR with the primers mk34 (SEQ ID NO: 2) and mk36 (SEQ ID NO:3). The backbone was amplified from pETDuet-1 via PCR with the primers mk32 (SEQ ID NO: 4) and mk33 (SEQ ID NO: 5). For the construction of pLD-o3 the antibiotic resistance gene of pJBEI-6409 was exchanged to a kanamycin resistance gene via Gibson Assembly. The insert was amplified via PCR from pET28a(+) with the primers mk42 (SEQ ID NO: 6) and mk44 (SEQ ID NO:7). The backbone was amplified via PCR from pJBEI-6409 with the primers mk20 (SEQ ID NO:8) and mk23 (SEQ ID NO:9). To delete the genes of the limonene-synthase and GPP-synthase from the assembled product, a PCR with the primers mk8 (SEQ ID NO: 10) and mk31 (SEQ ID NO:11) and a restriction digest with BamHI and self-ligation was done.
For the construction of pMK-24 the gene of the IPP-methyltransferase (SEQ ID NO: 1) was amplified via PCR with the primers mk52 (SEQ ID NO:12) and mk53 (SEQ ID NO:13) and pMK-17 as template. The PCR-product and the vector pET28a(+) were digested with NdeI and BamHI. The longer products of the digestions were ligated.
The sequences can be summarized in the following table:

| SEQ ID NO: | *Sequence* | *Sequence Description* |
|---|---|---|
| 1 | MSSEPTAALRTPLEQAAFDYYDNKRDDPINLKLGETDGFYHHHFAVGDFDRKVLLTSGEE RAQAINAELHRMETRQVDALSEALGPVAPQARILDGGSGRGGTAILLHKAFGCRVDGVNI SAYQNEFARRQAQEHNCAEKVHFHDRNMAATGFPDASFDYVVTNETTMYVDPYETFAEFA RLLKPAGRYVLLTWCNNDVLAPHPPEATAIDAHYHCHTHRRTTYLKALIESGLIPYQVDD LTQPATPYWELRSYSRYATGIEQHYLDGYRSDRVNYIRIASRRGAVPPSDTPTTAGAAAH | IPP-Methyltransferase of Streptomyces monomycini |
| 2 | TAACAATTCCCCTCTAGATAAGATTAAATAAGGAGGTTACCAATGTCCTCTGAGCCAACTGCTG | primer mk34 |
| 3 | AGCTCGAATTCGGATCCTTCAGTGGGCGGCGGCGCC | primer mk36 |
| 4 | AGGATCCGAATTCGAGCTC | primer mk32 |
| 5 | TGGTAACCTCCTTATTTAATCTTATCTAGAGGGGAATTGTTATCC | primer mk33 |
| 6 | ACAGGAGTCCAAGCGAGCTCAGGTGGCACTTTTCGGGG | primer mk42 |
| 7 | GGCACCGACGTCTTAATTAAAACAATAAAACTGTCTGCTTACATAAACAG | primer mk44 |
| 8 | GAGCTCGCTTGGACTCCT | primer mk20 |
| 9 | TTAATTAAGACGTCGGTGCCTAATGAGT | primer mk23 |
| 10 | CCTAAGGATCCAAACTCGAGTAA | primer mk8 |
| 11 | TATAGGATCCGTGCAGTCGGCGAA | primer mk31 |
| 12 | AGTCCATATGTCCTCTGAGCCAACTG | primer mk52 |
| 13 | AATTCGGATCCTTCAGTGGG | primer mk53 |

### Chemicals, media and bacterial strains

*E. coli* strains DH5α, MG1655 ΔendA ΔrecA (DE₃) and BL21(DE3) were grown in lysogeny broth (LB) medium (Bertani, 1951), terrific broth (TB) medium (Ausubel et al., 1994) and 2x YT medium (Ausubel et al., 1994) with 1% glycerol containing 100 µg/L ampicilin and/or 25 µg/L kanamycin, at 37 °C, respectively.

Solid medium was prepared by the addition of 1.5% agar-agar (m/v).

Antibiotics kanamycin and ampicilin were purchased from Carl Roth GmbH.

Solid-Phase Micro-Extraction (SPME) fibers were purchased from Sigma-Aldrich.

Standard compounds 5-methyl-isoprenol, 4-methyl-prenol, 4,4-dimethyl-prenol, 4,4-dimethyl-isoprenol and 4-methyl-geraniol were purchased from Enamine (Riga, Latvia); 4-methyl-isoprenol, Z and E configurations, were purchased from Akos (Steinen, Germany).

Protein cryo-stocks were kept at -80 °C in assay buffer containing 20% glycerol.

Lysis buffer (pH 7.4) consisted of 50 mM NaH₂PO₄, 300 mM NaCl, 10 mM imidazole and 5 mM DTT. Wash buffer (pH 7.4) consisted of 50 mM NaH₂PO₄, 300 mM NaCl, 250 mM imidazole and 5 mM DTT. Elution buffer (pH 7.4) consisted of 50 mM NaH₂PO₄, 300 mM NaCl, 250 mM imidazole and 5 mM DTT. Assay buffer (pH 6.7) consisted of 50 mM PIPES, 100 mM NaCl, 15 mM MgCl₂, and 5 mM DTT in ultra-pure water.

Glycerol, PIPES, NaCl, imidazole and NaH₂PO₄ were purchased from Carl Roth GmbH. DTT, acid phosphatase, IPP and S-Adenosyl-Methionine (SAM) were purchased from Sigma-Aldrich.

### Enzyme expression and purification

*E. coli* strain BL₂₁ (DE₃) was transformed with pMK-24. Pre-cultures were inoculated from cryo cultures into reaction tubes containing 5 ml LB medium with antibiotics and shaken overnight at 37 °C and 180 rpm. The 5 ml pre-cultures were then used to inoculate main cultures with 400 ml TB medium in 3 L shake flasks. After cultivation for 2 h at 37 °C (OD₆₀₀ of 0.6), IPTG at 0,1 mM was added for induction. Main culture was then cultivated at 18 °C, 110 rpm overnight.

Cells were harvested by centrifugation for 20 minutes at 4000 g and 4 °C, then re-suspended in 15 ml lysis buffer. Ultrasound sonication was performed on ice with 20% amplitude (0.5 seconds pulse, 1 second pause, 4 minutes pulse time), followed by digestion with RNAse (10 µg/ml) and DNAse (5 µg/ml) on ice for 15 min. After centrifugation for 20 minutes at 4000 g and 4°C, enzyme purification took place using His-Pur Ni-NTA Spin Columns (Thermo Fischer Scientific) according to the manufacturer's recommendations.

### In vitro incubations

Purified protein at 10 mM was added to 1 mL assay buffer containing 60 µM of IPP and 120 µM SAM. The enzymatic reaction was incubated at 30 °C overnight. Then 1 ml phosphatase solution (acid phosphatase 7.5 mg/ml in 0.1 M sodium acetate, pH 5.0) was added and the reaction was incubated at 30 °C for 2h. Following the reaction, volatiles in the headspace were extracted with SPME fiber.

### Production of alkylated isoprenoids

*E. coli* strain MG1655 ΔendA ΔrecA (DE3) was transformed with pLD-o3 and pMK-17. Pre-cultures were inoculated from cryo cultures into reaction tubes containing 5 ml LB medium with antibiotics and shaken overnight at 37 °C and 180 rpm. Main cultures with 15 ml 2XYT medium in 100 ml baffled shake flasks were inoculated from pre-culture to an OD₆₀₀ of 0.1. After cultivation for 2h at 37 °C (OD₆₀₀ of 1.0), IPTG at 0,1 mM was added for induction. Induced cultures had terpenes extracted after 24 hours of incubation at 30 °C and 180 rpm.

### HS-SPME-GC/MS analysis

Volatile compounds in the headspace of each assay were analyzed by extraction with an 85 µm SPME composed of PDMS and Carboxen (Stableflex). The SPME fiber was exposed in the assays' headspace for 30 minutes at 40 °C and then inserted into the injection port of a GC-MS-QP2010 (Shimadzu) containing a DB-5 (5%-phenyl)-methylpolysiloxane column with 30 m length and 0.25 mm thickness.

Measurements were conducted as follows: helium as carrier gas, splitless injections at 250 °C, 1 minute sampling time, and column flow of 1.1 ml/min. The column temperature was programmed as follows: 40 °C for 1.5 minute, 10 °C/min until 250 °C and then 20 °C/min until 300 °C.

Compounds in samples were identified by comparison of mass spectra fragment ions to purchased standards (see above: Standard compounds: 5-methyl-isoprenol, 4-methyl-prenol, 4,4-dimethyl-prenol, 4,4-dimethyl-isoprenol and 4-methyl-geraniol and 4-methyl-isoprenol (Z and E configurations) and isoprenol).

This HS-SPME-GC/MS analysis gave proof thereof that the analyzed compounds were identical to the purchased standard compounds. Those identified compounds are present in the in *vitro* assay and give proof thereof that isopentenyl-pyrophosphate is the substrate of isopentenyl-pyrophosphate-methyl transferase.

The following Table 1 shows the used plasmids in the described experimental section.

**Table 1: Used plasmids**

| Name | Description | Reference |
|---|---|---|
| pMK-24 | Vector for expressing the enzyme with SEQ ID NO 1 and N-terminal 6xHis-tag | |
| pET28a(+) | Source for kanamycin resistance gene for pLD-03 | Novagen No. 69864 |
| pETDuet-1 | Negative control and source for backbone of pMK-17 | Novagen No. 71146 |
| pJBEI-6409 | Expression vector for *E. coli* for the production of Limonene | (Alonso-Gutierrez et al., 2013) |
| pLD-03 | Expression vector for *E. coli* for the production of IPP and DMAPP, including the genes of acetoacetyl-CoA thiolase, HMG-CoA synthase, HMG-CoA reductase, mevalonate-5-kinase, phosphomevalonate kinase, mevalonate-5-pyrophosphate decarboxylase and isopentenyl pyrophosphate isomerase | |
| pMK-17 | Vector for *E. coli* expressing the enzyme with SEQ ID NO 1 | |

The following Table 2 shows the used primers in the described experimental section.

**Table 2: Used primers (overhangs in italic characters)**

| Name | Sequence |
|---|---|
| mk31 | *TATAGGATCCG*TGCAGTCGGCGAA (= SEQ ID NO:11) |
| mk32 | AGGATCCGAATTCGAGCTC (= SEQ ID NO:4) |
| mk33 | *TGGTAACCTCCTTATTTAATCTTA*TCTAGAGGGGAATTGTTATCC (= SEQ ID NO:5) |
| mk34 | 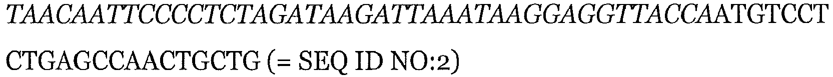 |
| mk36 | *AGCTCGAATTCGGATCCT*TCAGTGGGCGGCGGCGCC (= SEQ ID NO:3) |
| mk42 | *ACAGGAGTCCAAGCGAGCTC*AGGTGGCACTTTTCGGGG (= SEQ ID NO:6) |
| mk44 | 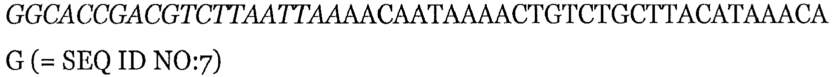 |
| mk8 | CCTAAGGATCCAAACTCGAGTAA (= SEQ ID NO:10) |
| mk20 | GAGCTCGCTTGGACTCCT (= SEQ ID NO:8) |
| mk23 | *TTAATTAA*GACGTCGGTGCCTAATGAGT (= SEQ ID NO:9) |
| mk52 | *AGTCCAT*ATGTCCTCTGAGCCAACTG (= SEQ ID NO:12) |
| mk53 | AATTCGGATCCTTCAGTGGG (= SEQ ID NO:13) |

The following Table 3 shows the used bacterial strains in the described experimental section.

**Table 3: Used bacterial strains**

| Name | Description | Reference |
|---|---|---|
| *E. coli* DH5α | F-, Φ80dlacZΔM15, Δ(lacZYA-argF)U169, deoR, recA1, endA1,hsdR17(rK-mK+), phoA, supE44, λ-, thi-1 | ATCC |
| *E. coli* MG1655 ΔendA ΔrecA (DE3) | F-, λ-, ilvG-, rfb-50, rph-1, ΔendA, ΔrecA | (Tseng et al., 2010) |
| E. coli BL21 (DE3) | F- *ompT gal dcm lon hsdS_{B}*(*r_{B}*-*m_{B}*-) λ(DE3 [*lacI lacUV₅-T7po7 ind1 sam7 nin5*]) [*malB*⁺]_{K- 12}(λ^{S}) | Novagen |

### REFERENCES

Alonso-Gutierrez, J., Chan, R., Batth, T.S., Adams, P.D., Keasling, J.D., Petzold, C.J., and Lee, T.S. (2013). Metabolic engineering of Escherichia coli for limonene and perillyl alcohol production. Metab. Eng. 19, 33-41.
Ausubel, F., Brent, R., Kingston, R., Moore, D., Seidman, J., Smith, J., and Struhl, K. (1994). Current Protocols in Molecular Biology, vol. 1, Wiley. N. Y.
Bertani, G. (1951). STUDIES ON LYSOGENESIS I. J. Bacteriol. 62, 293-300.
Dickschat, J. S., Nawrath, T., Thiel, V., Kunze, B., Müller, R., Schulz, S. (2007). Biosynthesis of the Off-Flavor 2-Methylisoborneol by the Myxobacterium Nannocystis exedens. Angewandte Chemie, International Edition, 46 (43), 8287-90.
Espah Borujeni, A., Channarasappa, A.S., and Salis, H.M. (2014). Translation rate is controlled by coupled trade-offs between site accessibility, selective RNA unfolding and sliding at upstream standby sites. Nucleic Acids Res., 42,2646-2659.
Gibson, D.G., Young, L., Chuang, R.-Y., Venter, J.C., Hutchison, C.A., and Smith, H.O. (2009). Enzymatic assembly of DNA molecules up to several hundred kilobases. Nat. Methods, 6, 343-345.
Komatsu, M., Tsuda, M., Omura, S., Oikawa, H. and Ikeda, H. (2008). Identification and functional analysis of genes controlling biosynthesis of 2-methylisoborneol. PNAS, 105 (21), 7422-7427.
Köksal, M., Chou, W. K., Cane, D. E., Christianson, D. W. (2012). Structure of geranyl diphosphate C-methyltransferase from Streptomyces coelicolor and implications for the mechanism of isoprenoid modification. Biochemistry, 51 (14), 3003-10.
Salis, H.M., Mirsky, E.A., and Voigt, C.A. (2009). Automated design of synthetic ribosome binding sites to control protein expression. Nat. Biotechnol. 27, 946-950.
Schmidt, A., Wächtler, B., Temp, U., Krekling, T., Seguin, A., Gershenzon, J. (2010). A bifunctional geranyl and geranylgeranyl diphosphate synthase is involved in terpene oleoresin formation in Picea abies. Plant Physiology, 152 (2), 639-55.
Tseng, H.-C., Harwell, C.L., Martin, C.H., and Prather, K.L. (2010). Biosynthesis of chiral 3-hydroxyvalerate from single propionate-unrelated carbon sources in metabolically engineered E. coli. Microb. Cell Factories, 9 (96).
Wang, C.-M., Cane, D. E. (2008). Journal of the American Chemical Society, 130 (28), 8908-9.

## Claims

1. A method for the production of isoprenoids by heterologous expression of a protein in a microorganism,
wherein, in said method, a protein is heterologously expressed as an alkyl transferase, wherein the protein is able to catalyze one or more alkylation(s) of isopentenyl-pyrophosphate.

2. The method of claim 1, wherein, in said method, the protein is heterologously expressed as an alkyl transferase and as an isomerase, wherein the protein is able to catalyze, additionally to the one or more alkylation(s) of isopentenyl-pyrophosphate, one or more isomerization(s) of isopentenyl-pyrophosphate or of alkylated isopentenyl-pyrophosphate.

3. The method according to any of the preceding claims, in which the heterologous expression is in Escherichia coli, Saccharomyces cerevisiae, Pichia pastoris, Bacillus subtilis, Corynebacterium glutamicum, Pfaffia rhodozyma, Methylobacterium extorquens, Pseudomonas putida, Cupriavidus necator, Rhodobacter sphaeroides, Streptomyces avermitilis, Streptomyces coelicolor or in Streptomyces monomycini.

4. The method according to any of the preceding claims, in which the heterologous expression is in Escherichia coli.

5. The method according to any of the preceding claims, wherein the protein is an isopentenyl-pyrophosphate-methyl transferase given by the SEQ ID NO. 1.

6. The method of any of the preceding claims, in which at least a further protein additionally to the alkyl transferase is expressed, which is able to catalyze the condensation of two or more substrates selected from the group consisting of isopentenyl-pyrophosphates, dimethylallyl-pyrophosphates, geranyl-pyrophosphates, farnesyl-pyrophosphates and geranylgeranyl-pyrophosphates, wherein at least one substrate of this further protein is an alkylated isopentenyl-pyrophosphate, an alkylated dimethylallyl-pyrophosphate, an alkylated geranyl-pyrophosphate, an alkylated farnesyl-pyrophosphate or an alkylated geranylgeranyl-pyrophosphate.

7. The method of any of the preceding claims, in which one or more other protein(s) additionally to the alkyl transferase is or are expressed, which is one or more protein(s) selected from the group consisting of phosphatase(s), isomerase(s), prenyltransferase(s) and synthase(s), wherein at least one substrate of the one or more other protein(s) is an alkylated isopentenyl-pyrophosphate, an alkylated dimethylallyl-pyrophosphate, an alkylated geranyl-pyrophosphate, an alkylated farnesyl-pyrophosphate or an alkylated geranylgeranyl-pyrophosphate.

8. The method of claim 7, in which the one or more other protein(s), which is or are expressed additionally to the alkyl transferase, is expressed as an isopentenyl-pyrophosphate-isomerase, which is able to catalyze the reversible isomerisation of 4-methyl-isopentenyl-pyrophosphate to 4-methyl-dimethylallyl-pyrophosphate.

9. The method of claim 7, in which the one or more other protein(s), which is or are expressed additionally to the alkyl transferase, is expressed as an isopentenyl-pyrophosphate-isomerase, which is able to catalyze the reversible isomerisation of 4-methyl-isopentenyl-pyrophosphate to 5-methyl-isopentenyl-pyrophosphate.

10. An alkyl transferase capable of catalyzing the one or more alkylation(s) of isopentenyl-pyrophosphate and optionally the one or more isomerization(s) of isopentenyl-pyrophosphate or alkylated isopentenyl-pyrophosphate, said alkyl transferase having an amino acid sequence represented by SEQ ID NO: 1.

11. A nucleic acid encoding the alkyl transferase according to claim 10.

12. A vector containing the nucleic acid according to claim 11.

13. A host cell containing the nucleic acid of claim 11 or the vector of claim 12.

14. An isoprenoid produced by the method according to any of claims 1 to 9.
